# EUROPÄISCHE PATENTSCHRIFT

(11) **EP 1 188 834 B1**
(45) Veröffentlichungstag und Bekanntmachung des Hinweises auf die Patenterteilung: **30.03.2005**
(21) Anmeldenummer: 01120658.8
(22) Anmeldetag: 30.08.2001
(51) Int. Cl.: C12N 15/86, A61K 39/00, C07K 14/71

(54) **Rekombinantes MVA mit der Fähigkeit zur Expression des HER-2/neu-Gens**
Recombinant MVA expressing the HER-2/neu gene
MVA recombinant capable d'exprimer le gène HER-2/neu

(30) Priorität: 30.08.2000 DE 10042598
(43) Veröffentlichungstag der Anmeldung: 20.03.2002
(73) Patentinhaber: GSF-Forschungszentrum für Umwelt und Gesundheit GmbH, 85764 Neuherberg (DE)
(72) Erfinder: Drexler, Ingo, Dr., 80797 München (DE); Erfle, Volker, Prof. Dr., 81679 München (DE); Sutter, Gerd, Dr., 80803 München (DE)
(74) Vertreter: Behnisch, Werner, Dr.

(56) Entgegenhaltungen:
- WO-A-00/20027
- WO-A-01/53463
- WO-A-97/02355
- SUTTER G ET AL: "NOVEL VACCINIA VECTOR DERIVED FROM THE HOST RANGE RESTRICTED AND HIGHLY ATTENUATED MVA STRAIN OF VACCINIA VIRUS" DEVELOPMENTS IN BIOLOGICAL STANDARDIZATION, BASEL, CH, Bd. 84, 1995, Seiten 195-200, XP000611390
- DREXLER I ET AL: "Modified vaccinia virus Ankara for delivery of human tyrosinase as melanoma-associated antigen: Induction of tyrosinase- and melanoma-specific human leukocyte antigen A*0201-restricted cytotoxic T-cells in vitro and in vivo." CANCER RESEARCH, Bd. 59, 1. Oktober 1999 (1999-10-01), Seiten 4955-4963, XP002185047
- CARROLL M W ET AL: "Highly attenuated modified vaccinia virus Ankara (MVA) as an effective recombinant vector: a Murine tumor model" VACCINE, BUTTERWORTH SCIENTIFIC. GUILDFORD, GB, Bd. 15, Nr. 4, 1. März 1997 (1997-03-01), Seiten 387-394, XP004094431 ISSN: 0264-410X

## Beschreibung

Die vorliegende Erfindung betrifft rekombinante MVA-Viren, die das menschliche HER-2/neu-Gen im MVA-Genom inseriert enthalten und exprimieren können, sowie die Verwendung dieser rekombinanten MVA-Viren für die Produktion von Polypeptiden, bspw. Antigenen oder Therapeutika, oder als Vakzine, oder für die Gentherapie.

### Hintergrund der Erfindung

HER-2/neu ist ein Selbst-Protein, das von einigen normalen Geweben exprimiert, von Krebszellen jedoch oft überexprimiert wird, und es wird als Folge einer Genamplifikation als Prototyp-Tumorantigen angesehen. Krebs, bei dem HER-2/neu-Überexpression erfolgt, umfasst Brust- und Ovarial-Karzinom (Slamon, D.J., Godolphin, W., Jones, L.A., Holt, J.A., Wong, S.G., Keith, D.E., Levin, W.J., Stuart, S.G., Udove, J., Ullrich, A., und Press, M.F. [1989]. Science 244: 707-12), Uterus-, Darm-Karzinom (Houldsworth, J., Cordon-Cardo, C., Ladanyi, M., Kelsen, D.P. und Chaganti, R.S. [1990], Cancer Res. 50: 6417-22) und Adenokarzinom der Lunge (Kern, J.A., Schwartz, D.A., Nordberg, J.E., Weiner, D.B., Greene, M.I., Torney, L., und Robinson, R.A. [1990], Cancer Res. 50: 5184-7). Die Existenz einer Immunität gegen HER-2/neu in Patienten mit Brustkrebs und die Fähigkeit, in Ratten eine Immunität gegen neu ohne eine wahrnehmbare Autoimmunität zu induzieren, eröffnen die Möglichkeit, HER-2/neu mit dem Ziel einer Tumorimmunität zu verwenden (Disis, M.L., Calenoff, E., McLaughlin, G., Murphy, A.E., Chen, W., Groner, B., Jeschke, M., Lydon, N., McGlynn, E., Livingston, R.B., Moe, R und Cheever, M.A., [1994], Cancer Res. 54: 16-20; Fisk, B., Blevins, T.L., Wharton, J.T und Ioannides, C.G. [1995], J. Exp. Med. 181: 2109-17; Kawashima, I., Tsai, V., Southwood, S., Takesako, K., Sette, A., und Celis, E., [1999], Cancer Res. 59: 431-435; Disis, M.L. und Cheever, M.A. [1998], Critical Reviews in Immunology 18: 37-45; Disis, M.L., Shiota, F.M. und Cheever, M.A. [1998], Immunology 93: 192-198).

Das Vaccinia-Virus, ein Mitglied der Gattung Orthopoxvirus in der Familie Poxviridae, wurde als Lebendvakzin zur Immunisierung gegen die menschliche Pocken-Erkrankung verwendet. Die erfolgreiche weltweite Impfung mit Vakzinia-Virus gipfelte in der Ausrottung des Variola-Virus, dem verursachenden Agens für Pocken (The global eradication of smallpox. Final report of the global commission for the certification of smallpox eradication. History of public Health, No. 4, Genf: World Health Organization, 1980). Seit dieser WHO-Erklärung wurde die Impfung universell eingestellt, ausgenommen für Personen, die gegenüber Pockenvirus-Infektionen gefährdet sind (Bspw. Laborarbeiter).

In letzter Zeit wurden Vaccinia-Viren ebenfalls zur gentechnologischen Manipulation viraler Vektoren für eine rekombinante Genexpression und für die potentielle Verwendung als rekombinante Lebendvakzine verwendet (Mackett, M., Smith, G.L. und Moss, B. [1982] P.N.A.S. USA 79, 7415-7419; Smith, G.L., Mackett, M. und Moss, B. [1984] Biotechnology and Genetic Engineering Reviews 2, 383-407). Hierbei werden DNA-Sequenzen (Gene), die für fremde Antigene codieren, mit Hilfe von DNA-Rekombinationstechniken in das Genom von Vaccinia-Viren integriert. Wird das Gen an einer Stelle in der viralen DNA integriert, die für den Lebenszyklus des Virus nicht essentiell ist, kann das neu produzierte rekombinante Vaccinia-Virus infektiös sein, d.h. es kann fremde Zellen infizieren und somit die integrierte DNA-Sequenz exprimieren (EP-Patentanmeldungen Nr. 83 286 und Nr. 110 385). Die derart hergestellten Vaccinia-Viren lassen sich zum Einen als Lebendvakzine für die Prophylaxe infektiöser Erkrankungen und zum Anderen zur Herstellung heterologer Proteine in eukaryotischen Zellen verwenden.

Das Vaccinia-Virus ist für Menschen infektiös, und nach der Impfung während der Pocken-Ausrottungs-Kampagne wurden gelegentlich schwere Komplikationen beobachtet. Den besten Überblick über das Auftreten von Komplikationen erhält man von einer nationalen Studie in den Vereinigten Staaten, welche die Impfung von etwa 12 Mio. Personen mit einem Vakzin überwacht, das auf einem Vaccinia-Virusstamm des New York City Board of Health beruht (Lane, J., Ruben, F., Neff, J. und Millar, J. [1969] New Engl. J. Med. 281, 1201-1208). Die aufregendste Möglichkeit zur Verwendung von Vaccinia-Vektoren für die Entwicklung rekombinanter Lebendvakzine wird durch Sicherheitsbelange und Richtlinien beeinflusst. Die meisten der in der Literatur beschriebenen Vaccinia-Viren beruhen auf dem Western-Reserve-Vaccinia-Virusstamm. Es ist dagegen bekannt, dass dieser Stamm eine hohe Neurovirulenz hat und sich daher für die Verwendung in Menschen und Tieren kaum eignet (Morita et al., Vaccine 5, 56-70 [1987]).

Für Vektoranwendungen würden die Gesundheitsrisiken durch Verwendung eines stark abgeschwächten Vaccinia-Virusstamms verringert. Es wurden speziell einige dieser Vaccinia-Virusstämme entwickelt, um ungewünschte Nebenwirkungen der Pockenimpfung zu vermeiden. Das modifizierte Vaccinia-Virus Ankara (MVA) wurde durch serielle Langzeit-Passagen des Ankara-Stammes des Vaccinia-Virus (CVA) auf Hühnerembryo-Fibroblasten erzeugt (für einen Überblick siehe Mayr, A., Hochstein-Mintzel, V. und Stickl, H. [1975] Infection 3, 6-14; Schweizer Patent Nr. 568 392). Das MVA-Virus wurde analysiert, um Veränderungen im Genom im Vergleich zum Wildtyp-CVA-Stamm zu bestimmen. Es wurden 6 Haupt-Deletionen der genomischen DNA (Deletion I, II, III, IV, V und VI) mit insgesamt 31000 Basenpaaren identifiziert (Meyer, H., Sutter, G. und Mayr, A. [1991] J. Gen. Virol. 72, 1031-1038). Das erhaltene MVA-Virus wurde gegenüber Vogelzellen stark Wirtszell-restringiert. MVA ist zudem durch seine extreme Abschwächung charakterisiert. Bei Untersuchungen in einer Reihe von Tiermodellen stellte sich heraus, dass MVA selbst in immunsupprimierten Tieren avirulent war. Noch wichtiger ist, dass die ausgezeichneten Eigenschaften des MVA-Stammes in ausgiebigen klinischen Untersuchungen gezeigt wurden (Mayr et al., Zbl. Bakt. Hyg. I, Abt. Org. B 167. 375-390 [1987], Stickl et al., Dtsch. med. Wschr. 99, 2386-2392 [1974]). Bei diesen Untersuchungen an mehr als 120 000 Menschen, einschließlich Hochrisiko-Patienten, ergaben sich keine Nebenwirkungen bei der Verwendung des MVA-Vakzins.

Es stellte sich heraus, dass die MVA-Replikation in menschlichen Zellen spät in der Infektion blockiert war, was den Zusammenbau zu reifen infektiösen Virionen verhindert. MVA konnte trotzdem virale und rekombinante Gene in hohen Mengen selbst in nicht-permissiven Zellen exprimieren, und es wurde vorgeschlagen, dass es als effizientes und ungewöhnlich sicheres Expressions-Vektorgen dient (Sutter, G. und Moss, B. [1992] Proc. Natl. Acad. Sci. USA 89, 10847-10851). Vor Kurzem wurden neue Vakzinia-Vektorsysteme auf der Basis von MVA entwickelt, bei denen fremde DNA-Sequenzen an der Deletionsstelle II im MVA-Genom oder im TK-Gen inseriert sind (Sutter, G. und Moss, B. [1995] Dev. Biol. Stand. Basel, Karger 84, 195-200 und US-Patent 5 185 146).

### Aufgaben der Erfindung

Aufgaben der vorliegenden Erfindung sind die Bereitstellung eines Expressionssystems auf der Basis eines rekombinanten MVA-Virus, welches menschliches HER-2/neu exprimieren kann, und Verfahren zur Herstellung der Polypeptide, bspw. Antigene oder Therapeutika, und zur Herstellung rekombinanter Vakzine, die auf diesem Expressionssystem beruhen.

### Lösung der Aufgaben

Diese Aufgaben werden erfindungsgemäß durch die Bereitstellung eines rekombinanten MVA gelöst, das DNA-Sequenzen zur Expression des HER-2/neu-Gens oder funktioneller Teile hiervon oder von Epitopen von HER-2/neu enthält. Weiterhin wird eine pharmazeutische Zubereitung bereitgestellt, welche das rekombinante MVA enthält, bevorzugt in Form einer Vakzine. In einer weiteren Ausgestaltungsform der Erfindung sind antigenpräsentierende Zellen (APCs) mit dem rekombinanten MVA infiziert.

Nachfolgend wird somit die Konstruktion und die Charakterisierung sowie die Verwendung eines rekombinanten MVA-Virus, das menschliches HER-2/neu exprimiert, zur Induktion HER-2/neu-spezifischer Immunantworten beschrieben.

Die Erfindung wird anhand von Figuren, einer allgemeinen Beschreibung sowie von Ausführungsbeispielen näher beschrieben. Die Erfindung ist jedoch nicht auf diese speziellen Ausgestaltungen beschränkt, sondern kann im Rahmen der Ansprüche Änderungen unterliegen.

Die anliegenden Figuren zeigen:
- Figur 1:: Die Konstruktion von rekombinantem MVA, das menschliches HER-2/neu exprimiert.
- Figur 2:: Die Charakterisierung von rekombinantem MVA in Bezug auf die Genexpression und das Wachstumsverhalten.
- Figur 3:: Die Charakterisierung von rekombinantem MVA in Bezug auf die genetische Stabilität und die Reinheit.
- Figur 4:: Eine FACS-Analyse der HER-2/neu-Expression durch mit rekombinantem MVA infizierte Zellinien.
- Figur 5:: Trennung lebensfähiger Zellen von apoptotischen Zellen.
- Figur 6:: Reife dendritische Zellen infiziert mit einer MOI=100 HER-2/neu Doppelfärbung optimales Gating 36 h nach der Infektion.
- Figur 7:: Reife dendritische Zellen infiziert mit einer MOI=100 HER-2/neu Doppelfärbung Gating apoptotischer DC's 36 h nach der Infektion.
- Figur 8:: Reifung unreifer muriner dendritischer Zellen, die mit rekombinantem MVA mit einer MOI von 5 infiziert wurden, wobei das MVA menschliches HER-2/neu MVA-huHer-2/neu P7.5 exprimiert. Die dendritischen Zellen wurden auf das frisch hergestellten, vom Knochenmark stammenden Zellen, die in vitro vier Tage lang mit murinem GM-CSF inkubiert wurden, erzeugt.

### Die vorliegende Erfindung

In einer Ausführungsform der Erfindung wird rekombinantes MVA bereitgestellt, das zumindest eine DNA-Sequenz enthält, die für das HER-2/neu-Protein codiert. Die Expression des HER-2/neu-Gens hat hierbei in solcher Weise zu erfolgen, daß z. B. nach Verabreichung an den Menschen, beispielsweise in Form einer Vakzine, die Induktion einer HER-2/neu-spezifischen Immunantwort bewirkt wird oder die Produktion rekombinanter HER-2/neu-Polypeptide erfolgt. Auch Abwandlungen des HER-2/neu-Gens, beispielsweise Teile des Gens, die in der Lage sind, eine derartige spezifische Immunantwort zu generieren, werden von der Erfindung mit umfaßt. Diese sind als "funktionelle Teile" bezeichnet. Hierbei handelt es sich insbesondere um solche DNA-Sequenzen, die für die extrazelluläre Domäne oder die intrazelluläre Domäne des HER-2/neu-Proteins codieren. Unter Abwandlungen des HER-2/neu-Gens sind auch solche DNA-Sequenzen zu verstehen, die zur Expression von Epitopen des HER-2/neu-Proteins befähigt sind und für diese Epitope codieren. Selbstverständlich sind von der Erfindung umfaßt auch DNA-Sequenzen, die für ein rekombinantes HER-2/neu-Protein codieren, welches sich von dem natürlich vorkommenden Protein durch Deletionen, Insertionen und/oder Aminosäurenaustausche unterscheidet, wobei jeweils die Funktion des HER-2/neu-Proteins beibehalten bleibt, insbesondere natürlich die Funktion, eine spezifische Immunantwort zu generieren.

Die Einführung der DNA-Sequenzen, die für das HER-2/neu-Gen oder funktionelle Teile hiervon codieren, wird mit Hilfe an sich bekannter DNA-Rekombinationstechniken durchgeführt, die eine Expression der Fremd-DNA durch den rekombinanten MVA-Vektor ermöglichen. Geeignete Klonierungstechniken stehen dem Fachmann zur Verfügung. Beispielsweise sei hingewiesen auf Sambrook, J. et al., Molecular Cloning: A Laboratory Manual, Cold Spring Harbor Laboratory, NY; oder Ausubel, F.M. et al., 1989, Current Protocols in Molecular Biology, John Wiley & Sons, New York, NY.

Die HER-2/neu-DNA-Sequenz wird an eine Stelle des MVA-Vektors eingeführt, die für den Lebenszyklus des Virus nicht essentiell ist, beispielsweise an die Stelle einer Deletion. Hierzu gehören die Deletionen I, II, III, IV, V, und VI, bevorzugt die Deletionen II und III (Meyer, H., Sutter, G. und Mayr, A., 1991, J. Gen. Birol. 72, 1031-1038). Das so hergestellte rekombinante MVA ist infektiös, d.h. es ist in der Lage, Fremdzellen zu infizieren und die integrierte Fremd-DNA-Sequenz HER-2/neu zu exprimieren. Derartige rekombinante Viren eignen sich als äußerst sichere Lebendvakzine zur Behandlung oder Prophylaxe von Tumoren, die im Zusammenhang mit der Expression von HER-2/neu stehe.

Das erfindungsgemäße rekombinante MVA kann, wie nachfolgend allgemein dargestellt, hergestellt werden. Eine detaillierte Beschreibung findet sich in den nachfolgenden Ausführungsbeispielen. Das Einführen des HER-2/neu-Gens in die MVA-DNA erfolgt beispielsweise im Wege einer homologen Rekombination, wobei die zu integrierende Sequenz an beiden Enden mit solchen Sequenzen versehen wird, die mit Sequenzen rekombinierbar sind, die den natürlich vorkommenden Deletionen, beispielsweise Deletion II oder III, im MVA-Genom benachbart angeordnet sind (Altenburger, W., Suter, C.P. und Altenburger J., 1989, Arch. Virol. 105, 15-27).

Ein derartiges DNA-Konstrukt, welches das HER-2/neu-Gen oder funktionelle Teile hiervon enthält, flankiert von MVA-DNA-Sequenzen, die benachbart von den Stellen der Deletionen, beispielsweise Deletion II oder III, im MVA-Genom vorkommen, werden in mit MVA infizierte Zellen eingeführt, um eine homologe Rekombination zu ermöglichen. Das eingeführte DNA-Konstrukt kann linear oder zirkulär sein. Bevorzugt wird ein zirkuläres DNA-Molekül, insbesondere ein Plasmid, eingesetzt.

Zur Expression der für HER-2/neu codierenden DNA-Sequenz sind regulatorische Sequenzen notwendig, die eine Transkription der DNA-Sequenz ermöglichen. Derartige regulatorische Sequenzen, beispielsweise Promotoren und Enhancer, sind an sich bekannt. Hierzu gehört beispielsweise auch das Vaccinia 11 kDa-Gen (vergleiche EP 0 198 328), das 7,5 kDa-Gen (vergleiche EP 0 110 385) oder der synthetische Promotor sP (vergleiche Sutter et al., Vaccine 1994, 12: 1032).

Das DNA-Konstrukt kann in MVA infizierte Zellen durch an sich bekannte Verfahren eingeführt werden, beispielsweise im Wege einer Transfektion durch Calciumphosphatpräzipitation (Graham et al., Virol. 52, 456-467, 1973; Wigler et al., Cell 777-785, 1979, durch Elektroporation (Neumann et al., EMBO J. 1, 841-845,1982), durch Mikroinjektion (Graessmann et al., Meth. Enzymology 101, 482-492, 1983), durch Liposomentechnologie (Straubinger et al., Methods in Enzymology 101, 512-527, 1983), durch Sphäroplastentechnologie (Schaffner, Proc. Natl. Acad. Sci. USA 77, 2163-2167, 1980) oder andere an sich bekannte Methoden. Bevorzugt wird die Calciumphophattechnik eingesetzt.

Nach Einführen des DNA-Konstrukts in die Eukaryontenzellen und nachher erfolgter Rekombination der HER-2/neu-DNA mit der viralen DNA kann das rekombinante MVA in an sich bekannter Weise isoliert werden, bevorzugt mit Hilfe eines Markergens (vergleiche Nakano et al., Proc. Natl. Acad. Sci. USA 79, 1593-1596, 1982; Franke et al., Mol. Cell. Biol. 1918-1924, 1985, Chakrabarti et al., Mol. Cell. Biol. 3403-3409, 1985, Fathi et al., Virology 97-105, 1986).

Das erfindungsgemäß hergestellte MVA kann ebenfalls ein Markergen tragen. Derartige Markergene erleichtern die Isolierung des rekombinanten Virus unter Zuhilfenahme an sich bekannter Techniken. Derartige Markergene sind an sich bekannt, und hierzu gehören Gene, die für Proteine wie β-Galactosidase, Neomycin, Alkoholdehydrogenase, Luciferase, Puromycin, Hypoxanthin-Phosphoribosyltransferase (HPRT), Hygromycin, sezernierte alkalische Phosphatase oder Grün- und Blau-Fluoreszenzproteine.

Selbstverständlich sind auch andere Methoden zur Herstellung des rekombinanten Virus der vorliegenden Erfindung möglich, beispielsweise die in nachfolgenden Ausführungsbeispiel näher beschriebene Klonierungsmethode.

Das modifizierte Vaccinia-Virus Ankara (MVA), ein stark abgeschwächter Vaccinia-Virusstamm mit eingeschränktem Wirtsspektrum, kann sich in Menschen und den meisten anderen untersuchten Säugerzelllinien nicht vermehren. Da jedoch die virale Genexpression in nicht-permissiven Zellen nicht beeinträchtigt ist, kann das erfindungsgemäße rekombinante MVA als ungewöhnlich sicherer und effizienter Expressionsvektor und rekombinantes Vakzin verwendet werden.

Bei einer erfindungsgemäßen Ausfuhrungsform wurden somit rekombinante MVA-Viren konstruiert, die die Expression des menschlichen HER-2/neu-Gens unter der Kontrolle des Vaccinia-Virus-Early/Late-Promotors P7.5 ermöglichen.

HER-2/neu kann Immunreaktionen induzieren, die das Tumorzellwachstum kontrollieren und Krebs, der mit HER-2/neu einher geht, heilen können.

In diesem Zusammenhang kann das rekombinante MVA-Virus, welches das HER-2/neu-Gen exprimiert, zur Immunisierung von Menschen für die Immuntherapie bei Krebspatienten verwendet werden.

Darüber hinaus kann das rekombinante MVA-Virus, welches HER-2/neu exprimiert, zur Bereitstellung von HER-2/neu an antigenpräsentierende Zellen, bspw. Dendritenzellen, Makrophagen und B-Zellen, die ebenfalls zur Immunisierung von Menschen für die Immuntherapie bei Krebspatienten verwendet werden, verwendet werden.

Das HER-2/neu exprimierende rekombinante MVA-Virus kann überdies zur Bereitstellung von HER-2/neu an antigenpräsentierende Zellen, bspw. Denditenzellen, verwendet werden, die wiederum ex vivo zur Erzeugung von Immuneffektorzellen verwendet werden, welche an Menschen zur Immuntherapie bei Krebspatienten abgegeben werden.

Das HER-2/neu exprimierende rekombinante MVA-Virus kann darüber hinaus zur Herstellung von rekombinantem HER-2/neu-Protein verwendet werden.

Die erfindungsgemäßen MVA-Vaccinia-Viren werden zur Herstellung von Vakzinen oder Therapeutika in eine physiologisch verträglich Form umgewandelt. Dies kann mit Hilfe der Erfahrung bei der Herstellung von MVA-Vakzinen erfolgen, die zur Impfung gegen Pocken verwendet werden (wie beschrieben von Stickl, H., et al. [1974] Dtsch. med. Wschr. 99, 2386-2392). Gewöhnlich werden etwa 10⁶ bis 10⁸ rekombinante MVA-Partikel in 100 ml phosphatgepufferter Salzlösung (PBS) in Gegenwart von 2% Pepton und 1% menschlichem Albumin in einer Ampulle, vorzugsweise einer Glasampulle, gefriergetrocknet. Das Lyophilisat kann Streckmittel (wie Mannitol, Dextran, Zucker, Glycin, Lactose oder Polyvinylpyrrolidon) oder andere Hilfsmittel (wie Antioxidantien, Stabilisatoren, usw.) enthalten, die sich zur parenteralen Verabreichung eignen. Die Glasampulle wird dann verschlossen, und kann dann, vorzugsweise bei Temperaturen unter -20°C, mehrere Monate lang aufbewahrt werden.

Zur Impfung oder zur Therapie kann das Lyophilisat in 0,1 bis 0,5 ml wässriger Lösung, vorzugsweise in physiologischer Kochsalzlösung, gelöst werden, und entweder parenteral, bspw. durch intramuskuläre Impfung, oder lokal, bspw. durch Impfung in einen Tumor oder an die Stelle eines Tumors, verabreicht werden. Erfindungsgemäße Vakzine oder Therapeutika werden vorzugsweise intramuskulär injiziert (Mayr, A. et al. [1978] Zbl. Bakt. Hyg., I. Abt. Orig. B 167, 375-390). Der Verabreichungsweg, die Dosis und die Anzahl der Verabreichungen kann durch den Fachmann auf bekannte Weise optimiert werden. Geeignetenfalls ist es angebracht, den Impfstoff mehrmals für einen längeren Zeitraum zu verabreichen, damit geeignete Immunantworten gegen das fremde Antigen erhalten werden.

Das nachstehende detaillierte Beispiel soll zu einem besseren Verständnis der vorliegenden Erfindung beitragen. Die Erfindung ist jedoch nicht auf das Beispiel beschränkt.

### Beispiele

### 1. Wachstum und Reinigung der Viren

### 1.1 Wachstum des MVA-Virus

Das MVA-Virus ist ein stark abgeschwächtes Vaccinia-Virus, das vom Vaccinia-Virusstamm Ankara (CVA) durch serielle Langzeit-Passagen auf primären Hühnerembryo-Fibroblasten-(CEF)-Kulturen hergeleitet ist. Für eine allgemeine Übersicht über die Geschichte der Produktion, der Eigenschaften und der Verwendung des MVA-Stammes kann auf die Zusammenfassung Bezug genommen werden, die von Mayr et al. in Infection 3, 6-14 [1975] veröffentlicht wurde. Aufgrund der Abschwächung in CEF repliziert das MVA-Virus in hohen Titern in dieser Vogel-Wirtszelle. In Säugerzellen ist das Wachstum von MVA jedoch stark gehemmt, und eine typische Plaquebildung durch das Virus ist nicht nachweisbar. Das MVA-Virus wurde daher auf CEF-Zellen gezüchtet. Zur Herstellung von CEF-Zellen wurden 11 Tage alte Embryos aus inkubierten Hühnereiern isoliert, die Extremitäten wurden entfernt, und die Embryos wurden zerkleinert und in einer Lösung, die aus 0,25% Trypsin bestand, 20 min lang bei 37°C dissoziiert. Die resultierende Zellsuspension wurde filtriert, und die Zellen wurden durch Zentrifugation bei 2000 U/min in einer Sorvall RC-3B-Zentrifuge 5 min bei Raumtemperatur sedimentiert, in 10 Volumina Medium A (MEM Eagle, bspw. erhältlich von Life Technologies GmbH Eggenstein, Deutschland) resuspendiert und erneut 5 min durch Zentrifugation bei 2000 U/min in einer Sorvall RC-3B Zentrifuge bei Raumtemperatur sedimentiert. Das Zellpellet wurde in Medium A, das 10 % fötales Kälberserum, (FCS), Penicillin (100 Units/ml), Streptomycin (100 mg/ml) und 2 mM Glutamin wieder aufgeschlämmt, so dass eine Zellsuspension erhalten wurde, die 500 000 Zellen/ml enthielt. Derart erhaltene CEF-Zellen wurden auf Zellkulturschalen verteilt. Diese wurden je nach gewünschter Zelldichte in Medium A in einem CO₂-Inkubator 1-2 Tage bei 37°C wachsen gelassen, und wurden entweder direkt oder nach einer weiteren Zellpassage für die Infektion verwendet. Eine eingehende Beschreibung der Herstellung von Primärkulturen befindet sich in dem Buch von R.I. Freshney, "Culture of animal cell", Alan R. Liss Verlag, New York [1983] Kapitel 11, S. 99ff.

MVA-Viren wurden wie folgt zur Infektion verwendet. Die CEF-Zellen wurden in 175 cm² Zellkulturflaschen gezüchtet. Bei 90 bis 100%iger Konfluenz wurde das Medium entfernt, und die Zellen wurden für eine Stunde mit einer MVA-Virussuspen-sion (0,01 infektiöse Einheiten (IU) pro Zelle, 0,02 ml/cm²) in Medium A inkubiert. Dann wurde mehr Medium A dazugegeben (0,2 ml/cm²), und die Flaschen wurden 2 bis 3 Tage bei 37°C inkubiert (bis etwa 90% der Zellen cytopathogene Wirkung zeigten). Rohe Virusstammpräparate wurden hergestellt, indem die Zell-Monolayer in das Medium geschabt und das Zellmaterial durch Zentrifugation bei 3000 U/min in einer Sorvall RC-3B Zentrifuge 5 min bei 4°C pelletiert wurden. Das rohe Viruspräparat wurde vor der weiteren Verarbeitung (z.B. Virusreinigung) bei -20°C aufbewahrt.

### 1.2 Reinigung der Viren

Die Reinigungsschritte, die unternommen wurden, um ein Viruspräparat zu erhalten, das so rein wie möglich war und keine für die Wirtszelle spezifischen Komponenten enthielt, ähnelten denjenigen, die von Joklik (Virology 18, 9-18 [1962]) beschrieben wurden. Rohe Virusstammpräparate, die bei -20°C aufbewahrt worden waren, wurden aufgetaut und einmal in PBS suspendiert (das 10 bis 20fache des Sedimentvolumens), und die Suspension wurde wie oben zentrifugiert. Das neue Sediment wurde im 10fachen Volumen Tris-Puffer 1 (10 mM Tris-HCl, pH-Wert 9,0) suspendiert, und die Suspension wurde kurz mit Ultraschall behandelt (Labsonic, L.B. Braun Biotech International, Melsungen, Deutschland; 2 x 10 sec bei 60 Watt und Raumtemperatur), um die Zelltrümmer weiter zu zerkleinern und um die Viruspartikel aus dem zellulären Material freizusetzen. Die Zell-. kerne und die größeren Zelltrümmer wurden in der nachfolgenden kurzen Zentrifugation der Suspension (Sorvall GSA-Rotor-erhältlich von DuPont Co., D-6353 Bad Nauheim, Deutschland, 3 min bei 3000 U/min und 10°C) entfernt. Das Sediment wurde noch einmal in Tris Puffer 1 suspendiert, mit Ultraschall behandelt und wie vorstehend beschrieben zentrifugiert. Die gesammelten Überstände, wurden vereinigt und über ein Kissen aus 10 ml 36%iger Saccharose in 10 mM Tris-HCl, pH-Wert 9,0 geschichtet und in einem Beckman SW 27/28-Rotor 80 min bei 13500 U/min bei 4°C zentrifugiert. Der Überstand wurde verworfen, und das Sediment, das die Viruspartikel enthielt, wurde in 10 ml 1 mM Tris-HCl, pH-Wert 9,0 aufgenommen, durch kurze Behandlung mit Ultraschall (2 x 10 sec bei Raumtemperatur, Gerät wie vorstehend beschrieben) homogenisiert und auf einen 20-40% Saccharosegradienten (Saccharose in 1 mM Tris-HCl, pH-Wert 9,0) zur weiteren Reinigung aufgetragen. Der Gradient wurde 50 min in einem Beckman SW41 Rotor bei 13000 U/min und 4°C zentrifugiert. Nach der Zentrifugation wurden getrennte Banden, die Viruspartikel enthielten, durch Pipettieren geerntet, nachdem das Volumen über der Bande abgesaugt wurde. Die erhaltene Saccharoselösung wurde mit drei Volumina PBS verdünnt, und die Viruspartikel wurden wiederum durch Zentrifugation (Beckman SW 27/28, 60 min bei 13500 U/min, 4°C) sedimentiert. Das Pellet, das nun überwiegend aus reinen Viruspartikeln bestand, wurde in PBS resuspendiert und auf Virus-Konzentra-tionen äquilibriert, die im Durchschnitt 1 bis 5 x 10⁹ IU/ml entsprachen. Die gereinigte Virus-Stammlösung wurde bei -80°C aufbewahrt und entweder direkt verwendet oder für die nachfolgenden Experimente mit PBS verdünnt.

### 2. Konstruktion und Charakterisierung rekombinanter MVA-Viren

### 2.1 Konstruktion und Charakterisierung des rekombinanten MVA-HER-2/neu-Virus

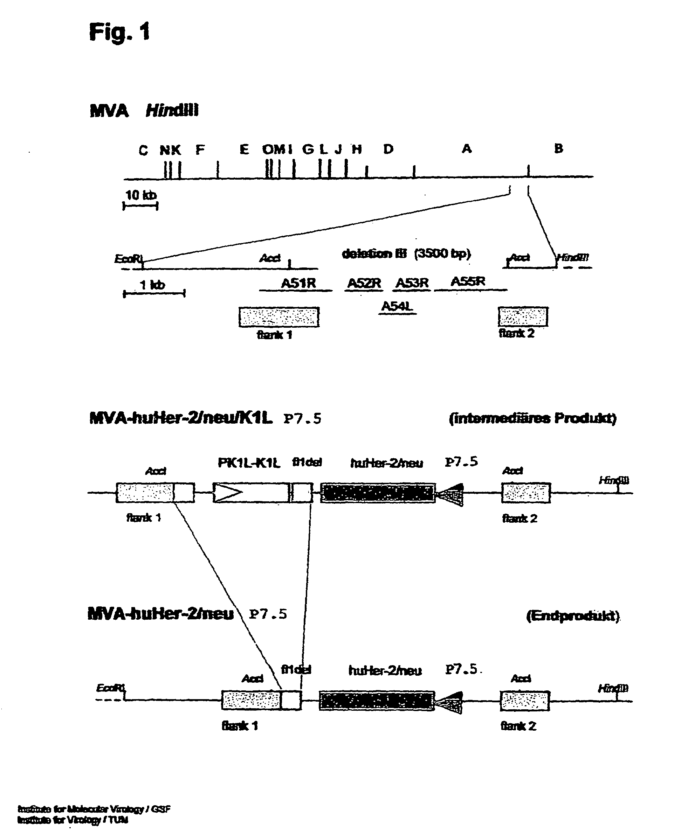

Ein 3845-bp DNA-Fragment, das das gesamte Gen des menschlichen HER-2/neu enthielt, wurde enzymatisch mit den Restriktionsenzymen Spe I und Sac I aus dem Expressionsplasmid pREP4/huHER-2/neu heraus geschnitten und in die pIIIdHR-P7.5-Stelle (Staib, C., Drexler, I., Ohlmann, M., Wintersberger, S., Erfle, V., Sutter, G., [2000] Biotechniques 28, 1137-1148) kloniert, so dass der Vektor pIIIdHR-huHER-2/neu [Figur 1] erhalten wurde. Dieses Plasmid kann zur Manipulation von rekombinantem MVA-Virus verwendet werden, welches das HER-2/neu-Gen unter der Kontrolle des Vaccinia Virus-Early/Late-Promoters P7.5 exprimiert.

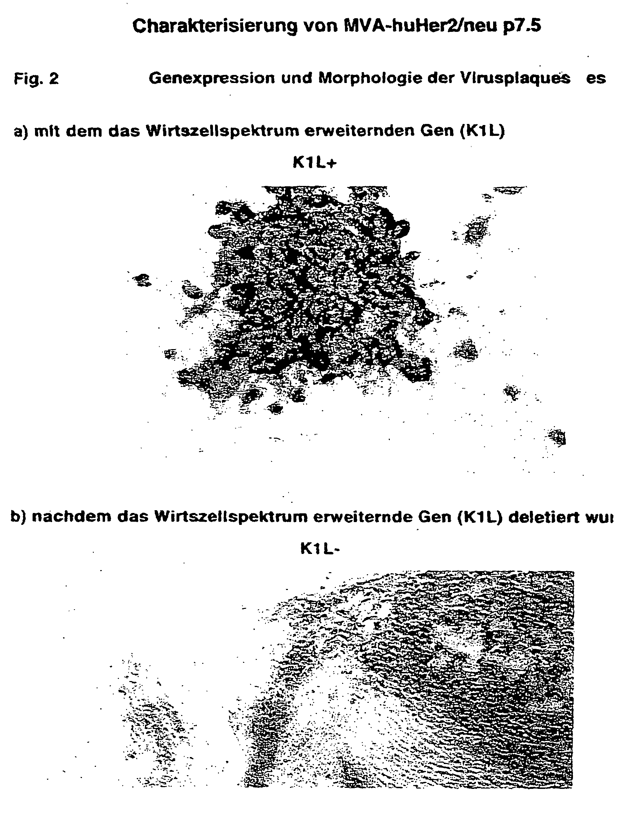

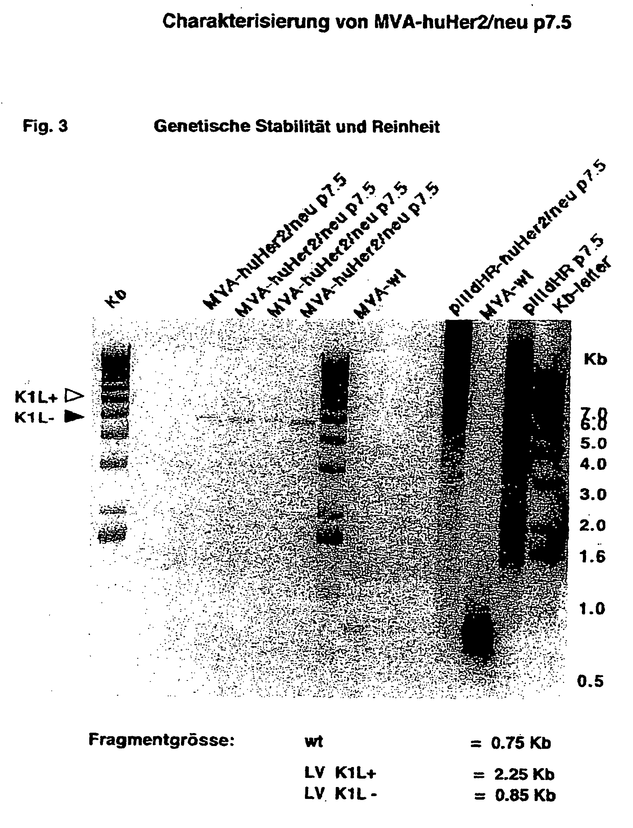

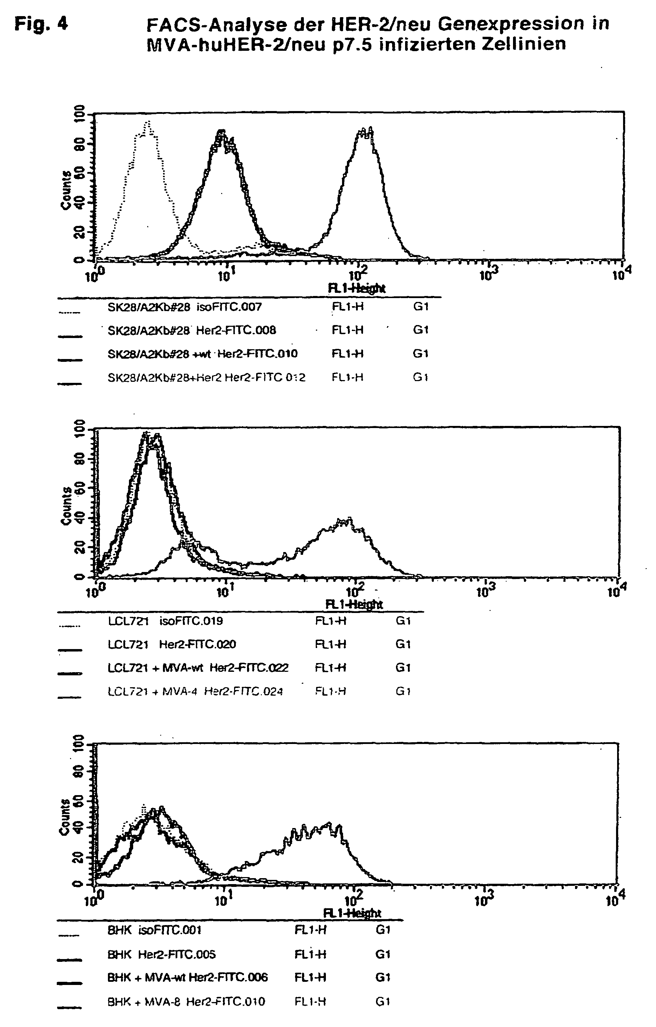

CEF-Zellen, die mit MVA mit einer Multiplizität von 0,05 TCID₅₀ pro Zelle infiziert wurden, wurden mit DNA des Plasmids pIIIdHR-huHer-2/neu wie beschrieben transfiziert (Drexler, I., Heller, K., Ohlmann, M., Erfle, V., Sutter, G., [1999] S. 57-73 in Methods in Molecular Medicine, Bd. 35: Gene Therapy: Methods and Protocols, Hrsg. W. Walther & U. Stein, Humana Press Inc. Totowa, NJ). Rekombinante MVA-Viren, die das HER-2/neu-Gen enthielten und das Vaccinia-Virus-Wirtsbereich-Markergen K1L coexprimierten, wurden durch aufeinanderfolgende Runden der Plaque-Reinigung in RK13-Zellen wie zuvor beschrieben selektiert (Staib, C., Drexler, I., Ohlmann, M., Wintersperger, S., Erfle, V., Sutter, G. [2000] Biotechniques 28, 1137-1148). Anschließend wurden rekombinante Viren durch Infektion von CEF-Monolayern amplifiziert, und die virale MVA-huHer-2/neu-DNA wurde durch PCR analysiert, um die genetische Homogenität des Virusstamms zu bestätigen [Figur 3]. Southern-Blot-Analysen von viraler DNA bestätigten die genetische Stabilität von MVA-huHer-2/neu und zeigten genau den Einbau des rekombinanten Gens an der Stelle von Deletion III im viralen Genom. Eine effiziente Expression von rekombinantem Protein wurde durch FACS-Analyse von permissiven und nicht-permissiven Zellen bestätigt, die mit MVA-huHer-2/neu (Figur 4) infiziert waren, sowie durch immunhistochemische Färbung von RK13- oder CEF-Zellen (Figur 2) mittels monoklonaler Mausantikörper gegen menschliches HER-2/neu (c-neu (Ab-2)(Calbiochem)).

Die Immunogenität von MVA-huHer-2/neu wurde durch Verwendung von Viruspräparaten als Lebendvakzine in A*0201/K^{b}-Tg-Mäusen gezeigt, die sowohl auf die zelluläre als auch auf die humorale Immunantwort zielen.

Eine HER-2/neu-spezifische CTL-Antwort kann durch das Vakzin in vivo nach der Immunisierung von A*0201/K^{b}-Tg-Mäusen induziert werden. A*0201/K^{b}-Tg-Mäuse wurden intraperitoneal (i.p.) entweder mit MVA-huHer-2/neu oder MVA-wt. geprimed. Milzzellen aus immunisierten Mäusen wurden mit dem A*0201-immunodominanten Her-2/neu-Peptid 369-377 in vitro restimuliert und auf eine HLA-A*0201-restringierte, HER-2/neu-peptidspezifische CTL-Antwort untersucht. Um zu zeigen, dass Antikörper gegen huHer-2/neu nach der Impfung induziert wurden, wurden Seren aus immunisierten Mäusen entnommen und gegen zelluläre Proteinlysate von huHer-2/neu-exprimierenden Zellen untersucht. Durch Nachweis HER-2/neu spezifischer Antikörper konnte die Induktion einer humoralen Antwort in den immunisierten Tieren gezeigt werden.

### 3. Infektion dendritischer Zellen mit dem rekombinanten MVA-HER-2/neu-Virus

Vaccinia-Viren (VV), die rekombinant für tumorassoziierte Antigene sind, wurden bereits erfolgreich eingesetzt, um eine protektive Tumor-Immunität in Mäusen zu induzieren. Die vorliegenden Studien fokussierten auf der Generierung von humanen, tumorreaktiven T-Zellen gegen HER-2/neu mit Hilfe von autologen dendritischen Zellen (DC), die mit dem modifizierten Vaccinia-Virus Ankara, rekombinant für HER-2/neu, (MVA-HER-2/neu), infiziert wurden.

Vor kurzem wurde jedoch beschrieben, daß eine VV-Infektion reifer dendritischer Zellen zu einer verminderten Expression von kostimulatorischen Molekülen an der Zelloberfläche im Sinne einer Umgehung des Immunsystems führt. Aus diesem Grund haben wir den Einfluß von MVA-HER-2/neu auf den Reifungsgrad von dendritischen Zellen anhand deren CD-Expressionsphänotyps untersucht.

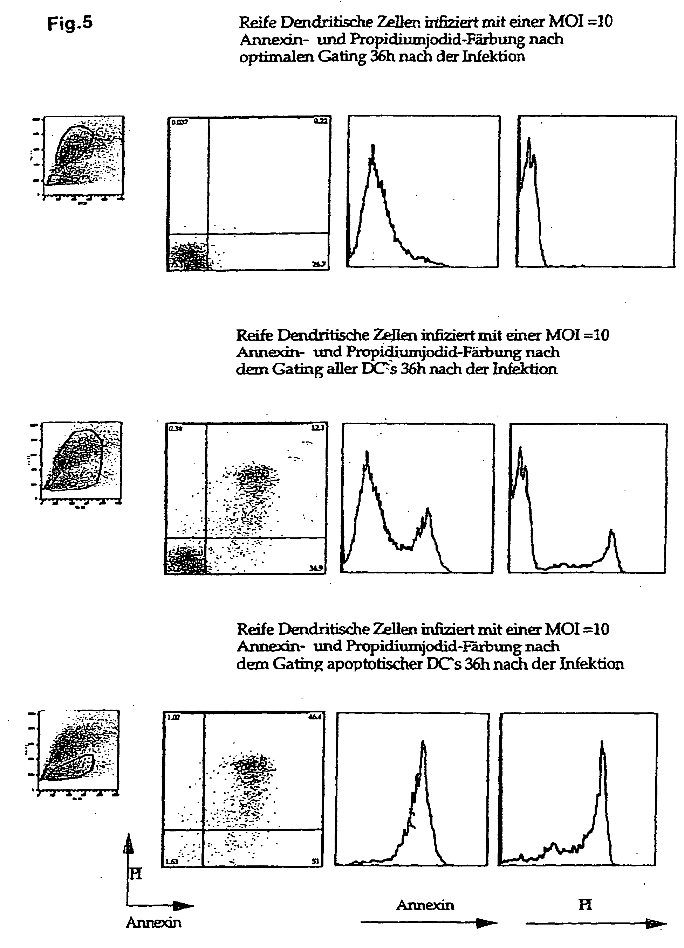

In FACS-Analysen wurde zuerst der Einfluß einer MVA-HER-2/neu-Infektion auf die Viabilität der infizierten DC's getestet. Mit Hilfe einer Annexin-Fitc und Propidium-Iodid-Färbung konnte gezeigt werden, daß infizierte Zellen nach einer gewissen Zeit apoptotisch werden und schließlich sterben. Da jedoch diese Vorgänge auch zu morphologischen Veränderungen führen, ist es möglich, durch optimales Gating, lebensfähige Zellen von apoptotischen zu trennen (siehe Figur 5).

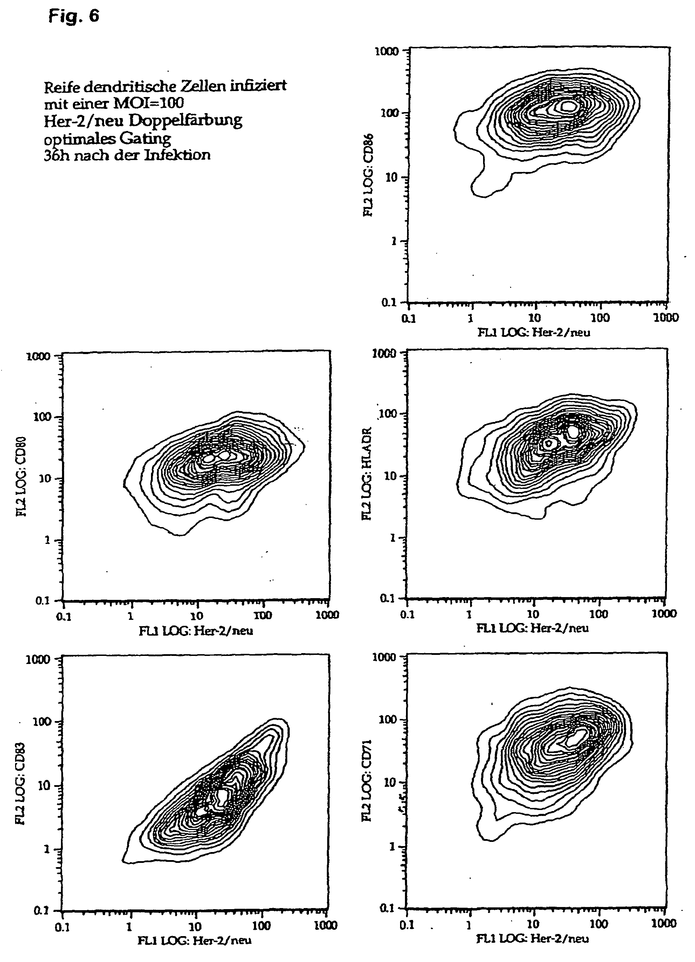

Zelloberflächenmoleküle auf DC's wie HLA-DR, CD83, CD80 und CD86, von denen bekannt ist, daß sie für das Priming von T-Zellen entscheidend sind, wurden im FACS nach verschiedenen Zeiten nach einer Infektion mit MVA-HER-2/neu analysiert. Um neben der Viabilität auch sicherzustellen, daß die untersuchten Zellen tatsächlich infiziert waren, wurde eine Doppelfärbung mit einem monoklonalen Antikörper gegen HER-2/neu durchgeführt. Hierbei zeigte sich, daß lebensfähige MVA-HER-2/neu infizierte DC's nicht ihre Oberflächenmoleküle nach unten regulierten, sondern im hohen Maße HLA-DR, CD83, CD80, CD86 als auch den Transferrin-Rezeptor CD71 exprimierten (siehe Figur 6). Der Prozentsatz HER-2/neu-positiver Zellen reichte von 50-95%, abhängig von der gewählten "Multiplicity of Infection".

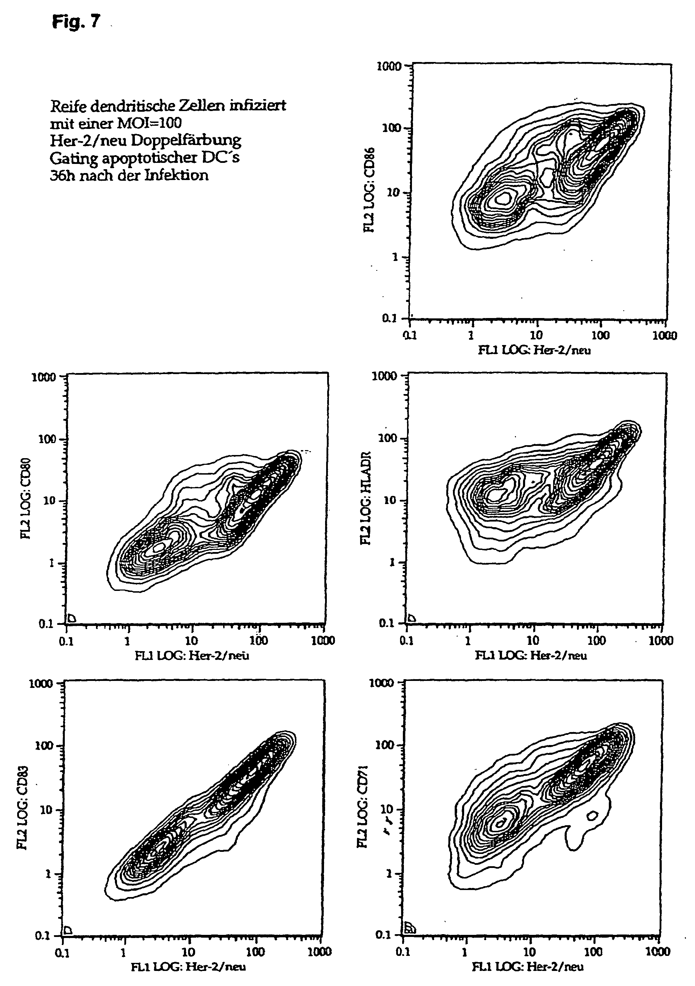

Etwa 36 h nach einer MVA-HER-2/neu Infektion gingen die meisten infizierten DC's in die Apoptose, jedoch konnten wir auch noch nach 60 h lebensfähige HER-2/neu exprimierende Zellen nachweisen. Apoptotische MVA-HER-2/neu infizierte DC's zeigten ein niedrigeres Niveau aller getesteten Oberflächenmoleküle als lebensfähige MVA-HER-2/neu infizierte DC's oder auch als nicht infizierte DC's (siehe Figur 7).

Schlußfolgernd kann man feststellen, daß ein Zeitraum von wenigstens 36 h, bis ein MVA-induzierter Zelltod im kritischen Maße eintritt, es den MVA-HER-2/neu infizierten DC's ermöglicht, T-Zellen erfolgreich zu primen.

Im Gegensatz zum Stand der Technik wurde damit überraschend festgestellt, daß die Infektion antigenpräsentierender Zellen, dargestellt am Beispiel reifer dendritischer Zellen, mit rekombinantem HER-2/neu-MVA zu einer hohen HER-2/neu-Expression in Zusammenhang mit kostimulatorischen Molekülen führt und nicht zu verminderter Expression letzterer.

Die Erfindung beschreibt somit rekombinantes MVA, welches DNA-Sequenzen enthält, die ein HER-2/neu-Protein oder -Polypeptid oder funktionelle Teile des Proteins oder des Polypeptids oder Epitope des HER-2/neu-Proteins oder -Polypeptids exprimieren können.

Die Erfindung beschreibt weiterhin eine pharmazeutische Zubereitung, insbesondere eine Vakzine, welche das rekombinante MVA-Virus enthält, Verfahren zur Herstellung des Virus, Verwendungen des Virus sowie Zellen, die mit dem Virus infiziert sind.

### 4. Verstärkte Ausreifung unreifer dendritischer Zellen nach Infektion mit MVA-buHER-2/neu

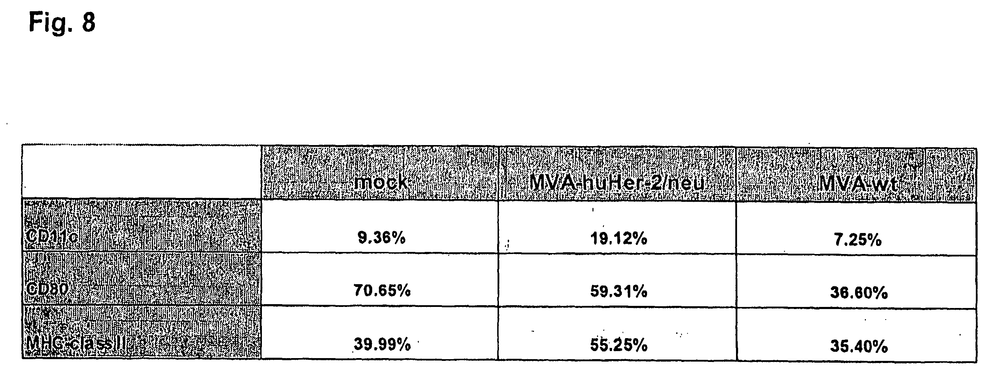

Nach Infektion noch unreifer dendritischer Zellen mit MVA-huHER-2/neu wurdeüberraschend eine verstärkte Ausreifung der infizierten Zellen festgestellt. Dieser Effekt konnte in der FACS-Analyse über eine Erhöhung der Anzahl von DC mit den typischen Oberflächenmarkern CD11c, CD80 und MHC-Klasse II-Molekülen gezeigt werden. Die Expression von CD11c und MHC-Klasse II-Molekülen nahm, absolut gesehen, zu, die Expession von CD80 nahm relativ zu MVA-Wildtyp (MVA-wt) infizierten dendritischen Zellen zu, da hier offensichtlich eine Abnahme der exprimierenden Zellen durch die Infektion zu beobachten ist (vgl. Figur 8).

Da insbesondere reife dendritische Zellen für die Antigen-Präsentation in einem immunogenen Kontext verantwortlich sind, scheint durch die Infektion mit MVA-huHER-2/neu ein positiver Effekt auf die Immunogenität des Antigens zu erfolgen, da mit der Infektion die Anzahl der professionellen Antigen-präsentierenden Zellen (APC) mit den zugehörigen kostimulatorischen Molekülen (wie z.B. CD80) und Antigen-präsentierenden Molekülen (wie z.B. MHC-Klasse II) erhöht wird. Durch die Expression von HER-2/neu wird also die Ausreifung der DC angeregt und die Anzahl von APCs erhöht.

### Methodik:

Die murinen DC wurden aus Knochenmarkszellen von Mäusen nach Ausschwemmen aus dem Femur gewonnen. Die Knochenmarkszellen wurden 4 Tage mit GMCSF inkubiert, was zur Generierung von unreifen DC führt, welche sich durch geringe Expression von Reifungsmarkern wie CD11c, CD80 und MHC-Klasse II -Molekülen auszeichnen.

Am Tag 4 wurde diese Zellpräparation der unreifen DC mit MVA-Viren mit einer MOI von 5 (5 infektiöse Einheiten pro Zelle) infiziert. Verwendet wurde MVA-huHer-2/neu P7.5 zu Expression von humanem Her-2/neu bzw. MVA-wt als Kontrolle. Die Zellen wurden hierfür in einem Gesamtvolumen von 100 µl mit den Viren für 1,5 h bei 37°C im Brutschrank inkubiert. Zusätzlich wurden Zellen nur mit Medium ohne Virus inkubiert (mock-infiziert). Danach wurde das Volumen mit frischem Medium auf 1 ml erhöht und die infizierten Zellen für weitere 16,5 h bei 37°C im Brutschrank inkubiert. Gesamtinkubationszeit 18h.

Nach 18 h wurden die infizierten und mock-infizierten Zellen für die Analyse der Expression folgender Moleküle an der Zelloberfläche mit den entsprechenden Antikörpern und Isotyp-kontrollen gefärbt:

| | |
|---|---|
| 1.) humanes Her-2/neu | |
| 2.) CD80 | (Reifungsmarker und kostimmulatorisches Molekül) |
| 3.) CD11c | (typischer Marker für DC) |
| 4.) MHC-Klasse II | (Reifungsmarker und antigenpräsentierendes Molekül) |

Zusätzlich wurde mit Propidiumjodid (PJ) eine Lebend/Tod-Färbung durchgeführt, um eine unspezifische Bindung der Antikörper durch tote Zellen (positiv für PJ) auszuschliessen. Es wurden damit nur lebendige Zellen analysiert. Die Ergebnisse sind in Figur 8 dargestellt.

## Patentansprüche

1. Rekombinantes MVA,
**dadurch gekennzeichnet, daß**
es DNA-Sequenzen zur Expression des HER-2/neu-Gens oder funktioneller Teile hiervon, die die Induktion einer HER-2/neu spezifischen Immunantwort bewirken, oder von Epitopen von HER-2/neu enthält.

2. Rekombinantes MVA nach Anspruch 1,
**dadurch gekennzeichnet, daß**
es das humane HER-2/neu-Gen enthält.

3. Rekombinantes MVA nach Anspruch 1,
**dadurch gekennzeichnet, daß**
es ein nicht-humanes HER-2/neu-Gen enthält.

4. Rekombinantes MVA nach Anspruch 3,
**dadurch gekennzeichnet, daß**
das nicht-humane HER-2/neu-Gen aus Maus, Ratte oder Kaninchen stammt.

5. Rekombinantes MVA nach einem oder mehreren der vorhergehenden Ansprüche,
**dadurch gekennzeichnet, daß**
als funktionelle Teile, die die Induktion einer HER-2/neu spezifischen Immunantwort bewirken, die DNA-Sequenzen zur Expression der extrazellulären Domäne oder der intrazellulären Domäne des HER-2/neu-Proteins enthalten sind.

6. Rekombinantes MVA nach einem oder mehreren der vorhergehenden Ansprüche,
**dadurch gekennzeichnet, daß**
die DNA-Sequenzen in nicht-essentiellen Bereichen, bevorzugt in Bereichen natürlich vorkommender Deletionen, im MVA-Genom integriert sind.

7. Rekombinantes MVA nach Anspruch 6,
**dadurch gekennzeichnet, daß**
die Stelle der natürlich vorkommenden Deletion die Deletion III oder eine andere Deletion in einem nicht-essentiellen Bereich des MVA-Genoms ist.

8. Rekombinantes MVA nach einem oder mehreren der vorhergehenden Ansprüche,
**dadurch gekennzeichnet, daß**
die DNA-Sequenzen unter Kontrolle des Vaccinia-Virus Early/Late Promoters P7.5 oder SP stehen und/oder unter Kontrolle weiterer, nicht Vaccinia-Virus eigener Promotor- und/oder Enhancer-Regulationssequenzen stehen.

9. Pharmazeutische Zubereitung,
**dadurch gekennzeichnet, daß**
sie wenigstens ein rekombinantes MVA nach einem oder mehreren der vorhergehenden Ansprüche 1-8 und pharmazeutisch verträgliche Träger- und Hilfsstoffe enthält.

10. Pharmazeutische Zubereitung nach Anspruch 9,
**dadurch gekennzeichnet, daß**
sie in Form einer Vakzine vorliegt.

11. Eukaryontenzelle,
**dadurch gekennzeichnet, daß**
sie mit einem rekombinanten MVA nach einem oder mehreren der Ansprüche 1-8 infiziert ist.

12. Eukaryontenzelle nach Anspruch 11,
**dadurch gekennzeichnet, daß**
sie eine antigenpräsentierende Zelle ist.

13. Eukaryontenzelle nach Anspruch 12,
**dadurch gekennzeichnet, daß**
sie eine dendritische Zelle, B-Zelle oder eine Makrophagenzelle ist.

14. Verwendung von rekombinantem MVA nach einem oder mehreren der Ansprüche 1-8 zur Herstellung eines Arzneimittels zur Therapie und Prophylaxe von Tumorerkrankungen.

15. Verwendung von rekombinantem MVA nach einem oder mehreren der Ansprüch 1-8 zur Herstellung einer Vakzine, zur Produktion von rekombinantem HER-2/neu-Protein oder zur Herstellung infizierter, das rekombinante Protein produzierender Eukaryontenzellen oder zur Verstärkung der Ausreifung unreifer dendritischer Zellen.

16. Verwendung einer pharmazeutischen Zubereitung nach Anspruch 9 oder 10 zur Herstellung eines Arzneimittels zur Immunisierung eines Tieres oder eines Menschen.

17. Verfahren zur Herstellung von rekombinanten HER-2/neu-Polypeptiden oder funktioneller Teile hiervon, die die Induktion einer HER-2/neu spezifischen Immunantwort bewirken, mit den nachfolgenden Schritten:
(a) Kultivieren von Zellen nach Anspruch 11 unter geeigneten Bedingungen, und
(b) Exprimieren, Isolieren und wahlweise Aufreinigen des vom HER-2/neu-Gen codierten rekombinanten HER-2/neu-Polypeptids.

18. Verfahren zur Herstellung von rekombinantem MVA mit den nachfolgenden Schritten:
(a) Einbringen des HER-2/neu-Gens oder funktioneller Teile hiervon, die die Induktion einer HER-2/neu spezifischen Immunantwort bewirken, in einen nicht-essentiellen Bereich eines MVA-Vektors zur Herstellung eines rekombinanten MVA-Vektors;
(b) Einbringen des rekombinanten MVA-Vektors und Vermehren des Vektors in einer Zelle; und
(c) wahlweise Isolieren der Virusteilchen.

## Claims

1. A recombinant MVA,
**characterized in that**
it contains DNA sequences for the expression of the HER-2/neu gene or functional portions thereof leading to induction of a HER-2/neu-specific immune response, or of epitopes of HER-2/neu.

2. A recombinant MVA according to claim 1,
**characterized in that**
it contains the human HER-2/neu gene.

3. A recombinant MVA according to claim 1,
**characterized in that**
it contains a non-human HER-2/neu gene.

4. A recombinant MVA according to claim 3,
**characterized in that**
the non-human HER-2/neu gene is derived from mouse, rat or rabbit.

5. A recombinant MVA according to one or more of the preceding claims,
**characterized in that**
as the functional portions leading to induction of a HER-2/neu-specific immune response the DNA sequences for the expression of the extracellular domain or of the intracellular domain of the HER-2/neu protein are contained.

6. A recombinant MVA according to one or more of the preceding claims,
**characterized in that**
the DNA sequences are integrated into non-essential regions, preferably in regions of naturally occurring deletions in the MVA genome.

7. A recombinant MVA according to claim 6,
**characterized in that**
the site of the naturally occurring deletion is deletion III or another deletion in a non-essential region of the MVA genome.

8. A recombinant MVA according to one or more of the preceding claims,
**characterized in that**
the DNA sequences are under the control of the vaccinia virus early/late promoter P7.5 or SP and/or under the control of other promoter and/or enhancer sequences not inherent to vaccinia virus.

9. A pharmaceutical preparation,
**characterized in that**
it contains at least one recombinant MVA according to one or more of the preceding claims 1-8 and pharmaceutically acceptable carriers and auxiliary agents.

10. A pharmaceutical preparation according to claim 9,
**characterized in that**
it is present in the form of a vaccine.

11. An eukaryotic cell,
**characterized in that**
it is infected with a recombinant MVA according to one or more of the preceding claims 1-8.

12. An eukaryotic cell according to claim 11,
**characterized in that**
it is an antigen-presenting cell.

13. An eukaryotic cell according to claim 12,
**characterized in that**
it is a dendritic cell, B cell, or macrophage cell.

14. The use of recombinant MVA according to one or more of the claims 1-8 for the preparation of a pharmaceutical composition for the therapy and prophylaxis of tumor diseases.

15. The use of recombinant MVA according to one or more of the claims 1-8 for the preparation of a vaccine, for the production of recombinant HER-2/neu protein or for the preparation of infected eukaryotic cells producing the recombinant protein or for enhancing the maturation of immature dendritic cells.

16. The use of a pharmaceutical preparation according to claim 9 or 10 for the preparation of a pharmaceutical composition for immunization of an animal or a human being.

17. A method for the preparation of recombinant HER-2/neu polypeptides or functional portions thereof leading to induction of a HER-2/neu-specific immune response, comprising the following steps of
(a) culturing cells according to claim 11 under appropriate conditions,
and
(b) expressing, isolating and optionally purifying the recombinant HER-2/neu polypeptide encoded by the HER-2/neu gene.

18. A method for the preparation of recombinant MVA comprising the following steps of:
(a) introducing the HER-2/neu gene or functional portions thereof leading to induction of a HER-2/neu-speicific immune response into a non-essential region of an MVA vector for the preparation of a recombinant MVA vector,
(b) introducing the recombinant MVA vector into and propagating the vector within a cell, and
(c) optionally isolating the virus particles.

## Revendications

1. MVA recombinant,
**caractérisé en ce que**
ou il comprend des séquences d'ADN pour l'expression du gène HER-2/neu ou de ses parties fonctionnelles qui provoquent l'induction d'une réponse immunitaire spécifique à HER-2/neu ou d'épitopes d'HER-2/neu.

2. MVA recombinant suivant la revendication 1,
**caractérisé en ce que**
il contient le gène HER-2/neu humain.

3. MVA recombinant suivant la revendication 1,
**caractérisé en ce que**
il contient le gène HER-2/neu non humain.

4. MVA recombinant suivant la revendication 3,
**caractérisé en ce que**
le gène HER-2/neu non humain provient de la souris, du rat ou du lapin.

5. MVA recombinant suivant l'une ou plusieurs des revendications précédentes,
**caractérisé en ce que**
il est contenu comme parties fonctionnelles qui provoquent l'induction d'une réponse immunitaire spécifique à HER-2/neu les séquences d'ADN pour l'expression des domaines extracellulaires ou des domaines intracellulaires de la protéine de HER-2/neu.

6. MVA recombinant suivant l'une ou plusieurs des revendications précédentes,
**caractérisé en ce que**
les séquences d'ADN sont intégrées dans des régions non essentielles, de préférence dans des régions de délétion se produisant naturellement, au génome MVA.

7. MVA recombinant suivant la revendication 6,
**caractérisé en ce que**
le point de la délétion se produisant naturellement est la délétion III ou une autre délétion dans une région non essentielle du génome MVA.

8. MVA recombinant suivant l'une ou plusieurs des revendications précédentes,
**caractérisé en ce que**
les séquences d'ADN sont sous le contrôle du promoteur P7.5 ou SP du Vaccinia-Virus Early/Late et/ou sous le contrôle d'autres séquences de régulation de promoteur et/ou d'activateur qui ne sont pas propres à Vaccinia-Virus.

9. Préparation pharmaceutique,
**caractérisée en ce qu'**
elle contient au moins un MVA recombinant suivant l'une ou plusieurs des revendications 1 à 8 précédentes, et un véhicule et un adjuvant acceptables pharmaceutiquement.

10. Préparation pharmaceutique suivant la revendication 9,
**caractérisée en ce qu'**
elle se présente sous la forme d'un vaccin.

11. Cellule eucaryote,
**caractérisée en ce qu'**
elle est infectée par un MVA recombinant suivant l'une ou plusieurs des revendications 1 à 8.

12. Cellule eucaryote suivant la revendication 11,
**caractérisée en ce que**
c'est une cellule présentant un antigène.

13. Cellule eucaryote suivant la revendication 12,
**caractérisée en ce que**
c'est une cellule dendritique, une cellule B ou une cellule macrophage.

14. Utilisation de MVA recombinant suivant l'une ou plusieurs des revendications 1 à 8, pour la production d'un médicament de thérapie et de prophylaxie de maladies tumorales.

15. Utilisation de MVA recombinant suivant l'une ou plusieurs des revendications 1 à 8, pour la production d'un vaccin, pour la production de la protéine d'HER-2/neu recombinant ou pour la production de cellules eucaryotes infectées produisant la protéine recombinante ou pour le renforcement de la maturation de cellules dendritiques immatures.

16. Utilisation d'une préparation pharmaceutique suivant la revendication 9 ou 10, pour la production d'un médicament d'immunisation d'un animal ou d'un homme.

17. Procédé de production de polypeptides de HER-2/neu recombinant ou de leurs parties fonctionnelles qui provoquent l'induction d'une réponse immunitaire spécifique à HER-2/neu, comprenant les stades suivants :
(a) culture de cellules suivant la revendication 11 dans des conditions appropriées, et
(b) expression, isolement et éventuellement purification des polypeptides de HER-2/neu recombinants codés par le gène HER-2/neu.

18. Procédé de production de MVA recombinant comprenant les stades suivants :
(a) introduction du gène HER-2/neu ou de ses parties fonctionnelles qui provoquent l'induction d'une réponse immunitaire spécifique à HER-2/neu dans une région non essentielle d'un vecteur MVA pour la production d'un vecteur MVA recombinant;
(b) introduction du vecteur MVA recombinant et multiplication du vecteur dans une cellule; et
(c) isolement des particules de virus.
